# EUROPEAN PATENT SPECIFICATION

(11) **EP 3 807 086 B1**
(45) Date of publication and mention of the grant of the patent: **11.10.2023**
(21) Application number: 19748593.1
(22) Date of filing: 12.06.2019
(51) Int. Cl.: B32B 5/02, B32B 7/02, B32B 7/06, B32B 27/12, B32B 29/02, B32B 29/06, B32B 5/26, B32B 15/085, B32B 15/09, B32B 15/20, B32B 37/00, B32B 41/00, B32B 7/025, B32B 7/04

(54) **AN ELECTROSTATICALLY LAMINATED NANOFIBRE COSMETIC COVER WITH A REMOVABLE LAMINATION LAYER, METHOD FOR ITS LAMINATION AND USE**
KOSMETISCHE ABDECKUNG AUS ELEKTROSTATISCH LAMINIERTER KOSMETISCHER NANOFASER MIT EINER ENTFERNBAREN LAMINIERSCHICHT, VERFAHREN ZU DEREN LAMINIERUNG UND VERWENDUNG
REVÊTEMENT COSMÉTIQUE DE NANOFIBRES STRATIFIÉ ÉLECTROSTATIQUEMENT COMPRENANT UNE COUCHE DE STRATIFICATION AMOVIBLE, SON PROCÉDÉ DE STRATIFICATION ET D'UTILISATION

(30) Priority: 12.06.2018 CZ 20182840
(43) Date of publication of application: 21.04.2021
(73) Proprietor: Nanopharma A. S., 53009 Pardubice (CZ)
(72) Inventor: BEREZKINOVA, Liliana, 10100 Prague (CZ); VYSLOU ILOVÁ, Lucie, 79811 Prostejov (CZ); KLÁP OVÁ, Andrea, 46800 Koberovy (CZ); RASSUSHIN, Vladimir, 250 72 Predboj (CZ); FOLBERGER, Ladislav, 16300 Prague 6, Repy (CZ)
(74) Representative: Sedlák, Jirí
(86) International application number: PCT/IB2019/054899
(87) International publication number: WO 2019/239337

(56) References cited:
- EP-A1- 1 535 846
- WO-A1-2017/034215
- FR-A2- 2 414 070
- US-A1- 2017 251 789

## Description

### Field of Technology

### Cosmetics, medicine, aesthetic medicine

### State of the Art

The cosmetic industry and the branch of aesthetic medicine have experienced big achievement recently. The quality of used raw materials and efficiency of dosage of active substances are the dominant aspects. The application of cosmetic preparations formulated as creams and gels has been established for long but it is not much efficient concerning effectiveness. The concentration of active substances in creams and gels is often up to 10 times higher than that required for the skin. The reason is inefficient resorbence of substances from creams or gels when the carrier, thus cream or gel forms a barrier for penetration of the active substance into skin.

In these, hurrying times, the users also appreciate simple cosmetic application or intervention. Cosmetic masks enjoy still growing popularity. Some of them can be applied on skin of face, or of neck or low neck, or other parts, as fluids. They usually are removed with water or peeled off after they get dry. Also face masks already pre-shaped start to appear on the market, that facilitate the application. Usually it is sufficient to place the mask on moist skin and let it work. Shaped masks usually are produced either of hydrophilic polymer or of nonwoven textiles, and they contain a broad range of active and supporting substances which affect appearance and vitality of skin. Transdermal application of active substances as mask presents several benefits: it eliminates a so called first pass effect, and thus it also improves the bioavailability of active substances, more uniform dosing of active components occurs against creams, and last but not least, the simplicity of the entire application and the increased user comfort should be mentioned.

The pre-shaped face masks are usually finished with thermal lamination. Lamination is necessary for handling individual masks and for cutting individual masks or parts from the sheet. Masks are covered with lamination polymer or inserted into a lamination pocket and laminated under temperature 80 to 120°C using heated drums. This process, however, destroys overwhelming majority of thermolabile active substances, for example vitamins, medicinal substances, plant extracts or colouring agents. Also thermal damage of lipid components of masks or damage of the carrying polymer can occur.

Development of nano-industries has brought many face nanomasks or nano-cover for aesthetic medicine in the market. Such a nanomask can be, for example, document US20100018641A1 describing a nanomask of hydrophilic polymer transformed in fibre together with substances with effect on skin vitality using electrostatic fibre spinning (electrospinning).

Document WO 2009133059 presents another example of a nanomask prepared using electrospinning. It describes preparation of a nanomask of branched polymer containing an active substance.

Document US8501642 describes preparation of nano-fibre paper, gels and emulsions of thermoplastic polymer, and such a nano-fibre composition can involve various natural substances and finds application also in the cosmetic as a carrier of active and natural substances.

Nano-fibre film according to document WO 2009022761 contains a broad range of drugs and can be used both in medicine and in cosmetics.

Document WO 2009133059 describes a nano-fibre matrix of branched polymer for active substances and controlled release of them.

Patent claim US 20160038611 describes a nano-fibre composition with bioactive components, mostly plant extracts (grape seeds, kiwi, broccoli, olive leaves, and the like) or natural products (honey, propolis and the like). Patent claim US 20100018641 describes a method of how to form a composite of nonwoven net for transport of substances positively influencing skin using electrospinning.

Documents EP 3 231 320 A1 and WO 2017/034 215 A1 describe a mask consisting of several, at least three, nanolayers deposited one on another. The mask according to that document is thermally laminated in the end of production.

The key disadvantage of all the nanomasks above is their high sensitivity to air moisture, ensuing from the nature of hydrophilic polymers and the content of active substances and very demanding handling of finished nanomasks due to thickness of the nanolayer, the appearance of which recalls a spider-web and it cannot be handled anyhow without being wrapped, otherwise it is damaged or impaired. The disadvantages above are so material that they completely prevent commercial production of such masks.

Therefore, it is necessary to form such a coating that protects the nanolayer but does not degrade active substances contained in it, contrary to thermal lamination. We have tested many active substances in the nanolayer that have survived the process of thermal lamination of the nanolayer, and only some 5 per cent of the original amount of active substances has been detected after thermal lamination.

### Description of the Invention

Electrostatically laminated nanofibre cosmetic cover was created which is advantageous to use as a cleansing or skin mask or generally, as a skin cover, with uniquely removable lamination layer that is necessary for protection of a fine nano-fibre layer applied on nonwoven fabric, where electrostatic lamination is carried as cold using only electrostatic forces, which does not harm active substances contained in the nano-fibre layer. Moreover, the application of this cover is very simple, the skin moisturizes, the lamination layer is stripped-off and the cover is applied. Active substances, if present, pass uniformly through the full area of application in moist environment directly into the skin. Electrostatically laminated nanofibre cosmetic cover can be used in many ways of application, also without active substances as cover only.

It was established that labile active substances encapsulated in fibres of a nano-fibre layer keep their natural properties and diffuse through skin membranes both after spinning and after lamination, which provides for transport of vitamins, medicinal substances and hydrating substances into cells of skin and thus, for the required effect of a facial mask.

Electrostatically laminated nanofibre cosmetic cover consists of an antistatic substrate layer of nonwoven fabric, of a nano-fibre layer of fibre-forming water-soluble polymer - carrier, and non-conducting synthetic lamination foil. The non-conducting synthetic lamination foil covers the nano-fibre layer and its adhesion to the nano-fibre layer is provided with orientation polarisation where in electric field dipoles of the material direct (turn) in the direction of that field and orientation polarisation of dielectric occurs. It concerns the nano-fibre layer and the lamination foil in this case.

Electrostatically laminated nanofibre cosmetic cover is specific due to the created electrostatic "layer" of oriented dipoles between the nano-fibre layer or the layer of antistatic nonwoven fabric and the lamination foil, the aim of which is to generate electrostatic potential between layers of the nanomask, or cover, where the lamination foil adheres easily to the outstanding part of the nanomask and then it separates easily from it and, above all, from the nano-fibre layer and therefore, the nano-fibre layer keeps fully compact without any mechanic damage. Thanks to the cold lamination, degradation of active components in the nano-fibre layer does not occur. As well as omission of pressure lamination keeps all the physical properties of the nano-fibre layer important for the right function of cosmetic cover, thus for its defined dissolution and release of active substances, if required.

The substrate layer of nonwoven fabric has antistatic finish and it is advantageous if it is produced of polypropylene or polyamide. It is advantageous if the non-conducting synthetic lamination foil is produced of polyamide or silicone paper.

The nano-fibre layer is produced of fibre-forming water-soluble polymer, namely itself or in mixture, selected in this group: polyvinyl alcohol, polyvinyl acetate, polycaprolactone, polydioxanone, polyethylene oxide, polyhydroxibutyrate, polyvinyl butyrate, polyvinyl butyral, polylactide, polylactide-co-caprolactone, polylactide-co-glycolide, polylactide-co-caprolactone-co-glycolide, polyvinyl alcohol, polyvinyl pyrrolidone, cellulose acetate, sodium alginate, elastin, collagen, hyaluronic acid, zein, gelatine, silk, chitosan, carboxymethyl chitosan, chitin, wheat protein, hyaluronan, glucans, and other polysaccharides and their copolymers themselves, and combined with polyvinyl alcohol. It is advantageous if the nanofibre layer is produced of polyvinyl alcohol (PVA), hydrated polyvinylacetate or of polysaccharide or their mixture.

A nano-fibre layer of water-soluble polymer is prepared using electrostatic fibre spinning and it is advantageous if it has specific weight 1.5 to 2.5 g/m², and it is advantageous if it contains 0.001 to 25 weight per cent of active substance, or their mixture. It is quite evident that various active substances can be added into the fibre-forming PVA solution - this is the state of the art. Therefore, we have always documented one representative of active substances of that group in the examples. Experts skilled in the art will find evident that similar active substances and/or their combination will operate similarly.

The active substances with relative shares are as follows:
0.01 to 10 wt.% of vitamin C, of mineral salts of ascorbic acid, and it is advantageous if 1 to 7 wt.%, and 4 to 6 wt.%, respectively;
0.01 to 10 wt.% of vitamin A, retinyl palmitate, ester of retinol and of palmitic acid, of their derivatives and salts, and it is advantageous if 0.1 to 6 wt.%, and 1 to 3 wt.%, respectively;
0.01 to 10 wt.% of vitamin E, ester of acetic acid and of tocopherol, of their derivatives and salts, and it is advantageous if 0.5 to 7 wt.%, and 2.5 to 5 wt.%, respectively;
0.01 to 5 wt.% of panthenol, alcohol analogues of pantothenic acid, and it is advantageous if 0.1 to 3 wt.%, and 0.5 to 1 wt.%, respectively;
0.001 to 3 wt.% of vitamin B3, niacin amide, and it is advantageous if 0.01 to 2 weight %, and 0.03 to 0.5 wt.%, respectively;
0.001 to 3 wt.% of vitamin B6, pyridoxine, and it is advantageous if 0.01 to 2 wt.%, and 0.03 to 0.5 wt.%, respectively;
0.001 to 3 wt.% of riboflavin, sodium phosphate, of its derivatives or salts, and it is advantageous if 0.01 to 2 wt.%, and 0.03 to 0.5 wt.%, respectively;
0.01 to 10 wt.% gluconolactone, and it is advantageous if 1 to 8 wt.%, and 3 to 6 wt.%, respectively;
0.001 to 3 wt.% of hyaluronic acid, its salts or derivatives, and it is advantageous if 0.01 to 2 wt.%, and 0.03 to 0.5 wt.%, respectively;
0.01 to 8 wt.% of arbutin, of its derivatives, and it is advantageous if 0.1 to 5 wt.%, and 1 to 3 wt.%, respectively;
0.01 to 12 wt.% of natural extracts, and it is advantageous if this is extract of *Fucus vesiculosus*, and it is advantageous if 0.1 to 8 wt.%, and 0.5 to 4 wt.%, respectively; and any mixtures thereof.

Further, the active substances given below can be used in concentration 0.1 to 10 wt.% (unless specified otherwise):
active substances against acne, and it is advantageous if it is azelaic acid, salicylic acid, benzoyl peroxide;
active substances against ageing, and it is advantageous if it is maltobionic: acid, N-acetyl tyrosinamide;
antioxidation active substances, and it is advantageous if 10 to 25 wt.% of rose extract; calming active substances, and it is advantageous if ethylferulate;
hydrating active substances, and it is advantageous if hydroxymethylurea, extract of *Malva Sylvestris;*
moisturing substances, and it is advantageous if extract of *Cnidium Monnieri*, Ajidew^{®};
skin conditioners, and it is advantageous if methylprotocatechuic acid, cetearyl ethylhexanoate;
antiinflammatory active substances, and it is advantageous if bisabolol or sodium fluoride; revitalising substances, and it is advantageous if collagen;
skin nutrients, and it is advantageous if vitamin B1, its derivatives and salts;
antibacterial active substances, and it is advantageous if triclosan;
antistatic active substances, and it is advantageous if cetrimonium chloride or quarternary ammonium compounds;
astringent active substances, and it is advantageous if extract of *Ilex paraguariensis*, extract of *Verbascum thapsus*, extracts of citrus fruits;
whitening active substances, and it is advantageous if Lumiskin^{™}, extract of *Phytexcell mulberry*, ascorbyl glucoside;
cleansing active substances, and it is advantageous if sodium cocoyl isenthionate, cocamidopropyl dimethylamine;
refracting active substances, and it is advantageous if function lipids;
refreshing active substances, and it is advantageous if extract of *Chamomilla recutita*;
active skin substances, and it is advantageous if root juice of *Polymnia sonchifolia*, dimethicone;
active substances for skin protection, and it is advantageous if tocopherol esters, mineral salts of ascorbic acid, extract of *Laminaria digitate*;
smoothing active substances, and it is advantageous if biotin, extract of *Olea Europaea* fruit; tanning active substances, and it is advantageous if erythrulose, stearate;
tinting active substances, and it is advantageous if extract of *Chamomilla recutita* flowers; UV-protective active substances, and it is advantageous if ferulic acid, zinc oxides in cocos alkanes (UV Cut ZnO-72-VL); and any mixtures of them.

A method of electrostatic lamination was developed concurrently, it allows conservation of cosmetic and aesthetic products without using heat and this way loss of thermolabile active substances and provides for mechanic protection of the nano-fibre layer.

The development of the nanomask has stressed simple application and also efficient incorporation of active substances (AL) into upper skin layers - epidermis and dermis. As the skin is the natural barrier of the body, only a limited amount of substances from cosmetic products penetrates into it. The nanomask has been developed so that it would dissolve on moistened skin to create environment suitable for transferring the active ingredients from a water-soluble polymer, preferably polyvinyl alcohol (PVA), hydrated polyvinyl acetate, polysaccharide or the like, into the skin. This is achieved using techniques for production of the nanomask which is produced using electrostatic fibre spinning from solution, and the solution contains water-soluble polymer and an active substance or their mixture. The conditions for forming fibres of polymer solution under action of strong electric field eliminate bacterial or microbiological contamination. Therefore, the formulation of the nanomask allows to omit cosmetic additives, like filling, stabilising and conserving agents.

### Preparation of nano-fibre layer of water-soluble carrier

Water-soluble polymer is dissolved at temperature 70 to 99°C and under continuous stirring in demineralized water in a homogeneously heated and homogeneously stirred reaction vessel and a solution is formed, it is advantageous if it is 10 wt.%. After complete dissolution of the polymer, the solution is cooled homogeneously to temperature 25°C. It is advantageous if active substances are added to the solution at this moment. The solution of polymer and of active substances, if any, is spun electrostatically and applied on substrate nonwoven fabric. It is advantageous, if the nonwoven fabric is produced of polypropylene or polyamide, and the nano-fibre layer is applied on the nonwoven fabric with specific weight 1 to 10 g/m². The specific weight is controlled by samples taking. The thickness of the nanofibres spun under such conditions is 100 to 300 nm, and it is advantageous if 250 ± 70 nm.

It is advantageous if electrostatic fibre spinning runs under the following conditions: the fibre-forming electrode is a string 0.3 mm thick with a die 0.6 to 0.7 mm and a gap between the electrode and the counter-electrode set to 130 up to 190 mm, frequency of move of the rider and the cartridge with the fibre-forming solution is 3-5 s, voltage applied at the fibre-forming electrode is +30 to 60 kV, voltage applied at the counter-electrode (collector) was -20 to 30 kV, draw-off speed of substrate nonwoven fabric 8 to 15 mm/min. The temperature inside the fibre-forming chamber is 20 to 25°C and air moisture is 30 to 40%.
nonwoven fabric can be produced of almost any material, because it serves as substrate for nano-fibre layers application only.

It was proven that it is possible to make fibres of solutions of water-soluble carrier with different concentrations and a homogeneous nano-fibre layer forms on substrate nonwoven fabric. Preparation of solution of carrier for fibre-forming is the key step because this solution must be highly homogeneous. Should the solution be inhomogeneous, defects will appear in the nano-fibre layer, for example drop-shaped ones presented in Figures 3 and 4 caused by wrong heating of the solution and its imperfect mixing.

In electrostatic field under voltage in order of tens of kilovolts, the prepared solution is spun and the solvent, water, is separated (vaporised). Nanofibres are caught on substrate nonwoven fabric, and if they are in higher volume they form a compact layer, in order of units g/m². The substrate nonwoven fabric provides for mechanic properties and support for produced layer of nanofibres. The carrier with nano-fibre layer of polymer or of polymer and active substance is wound into a roll which is further processed in the next technological step via lamination and cutting. The formed layer with the substrate nonwoven fabric is formed into a shape of a skin mask or other special shapes for local application.

The benefit of the nanomasks presented is their better adhesion to cutis or skin, thanks to small size of fibres and filling of all folds and humps in cutis and skin for example next to the nose.

The indisputable benefit of the nanomask also is the fact that active substances are "closed" into fibres of the water-soluble polymer carrier and they are released in application of the nanomask on moistened cutis or skin. Lamination of nanomask is essential for further manipulation with the nanomask.

The fibre-forming solution contains 75 to 92 wt.% of deionized water, and it is advantageous if 85 to 90 wt.%. Further, it is advantageous if the fibre-forming solution contains 0.1 to 1 wt. % of conservative agents, and even better if 0.15 to 0.8 wt.%. It is advantageous if the conservative agents are represented by fenoxyethanol, benzyl alcohol, ethylhexylglycerin and other substances.

In case of fibre spinning of active substances poorly soluble in water it is advantageous if a surfactant is used for preparation of the fibre-forming solution, and it is advantageous if it is represented by Kolliphor 40D and other substances.

Tests presented in example 6, documented that over 60 % of the total transported active substances passes through an experimental nitrocellulose membrane during the first 5 minutes, and it is 98% in 15 minutes. Another experiment (example 7) checked that the application of nano-fibre cover containing active substances is more efficient than application hydrogel - up to 270% of the substance was transported against transport of active substances from hydrogel during 14 hours of releasing active substances from nano-fibre cover into skin. These results indicate nanofibre cover for efficient and user friendly application with actual entry of active substances into skin.

Electrostatic lamination was developed after long-lasting problems to keep efficient concentration of active substances in produced nanomasks. Analysis of nanomasks after the classic lamination showed that there is less than 5% of the initial concentration of active substances in the finished thermally laminated product. A method was searched of how to avoid thermal lamination while keeping the properties of a laminated product. After fortuitous application of polyamide fabric to a produced nanomask in environment with decreased air moisture, electrostatic forces caused relatively strong adhesion of the fabric to the nanomask. Therefore, we started testing of electrostatic force of various magnification and options of how to generate charge. The ideal charge showed to be 20 to 30 kV generated by a charge generator for single-layered nanomasks, and charges up to 30 kV can be used for masks of higher specific weight than 4 g/m². Concerning production, it is advantageous to use polyamide fabric with positive charge combined with a nanomask applied on nonwoven fabric with negative charge. Concerning technique, it is possible to reverse the charge, thus to use polyamide fabric with negative charge and nonwoven fabric with positive charge. Draw-off speed of the laminated nano-fibre cover, thus lamination speed, is 2 to 5 m/min.

The benefit of the solution presented of electrostatic lamination is its simplicity, low finance demands and applicability to all the current types of masks and cover, and possibly to other cosmetic and aesthetic products containing thermolabile components.

The lamination principle applies the Coulomb law in electric field passing dielectric which is represented by two isolation layers, one layer is the fabric, and the other layer are PVA nanofibres.

The equipment for cold electrostatic lamination consists of a D.C. high voltage generator and a charging rod of the generator, or an electrode. Smoothed layers of a nano-fibre sheet and polyamide fabric pass electric field where positive atom nuclei move along field lines, negative shells deform in the opposite direction, electric dipoles form, the effect is called atom polarisation of dielectric. In electric field, the dipoles direct along the electric field (they turn into the direction), orientation polarisation of dielectric occurs.

The definition of homogeneous field necessary to achieve the required adhesion of both connected materials is completed with the corresponding magnification of specific resistance of the layer so that the adhesion effect is uniform on the whole area of the connected materials.

For nonwoven PA fabric, specific resistance is 1013 'Ω cm for a PVA nano-fibre layer 108 'Ω cm.

Other parameters that affect stability and homogeneity of electrostatic charge are relative air moisture and speed of sandwich movement (3.5 m/min.). Parameters of charging generator for the nano-fibre layer with specific weight 2g/m²: voltage - up to 30 kV, electric current up to 5 mA.

Therefore a drying element (silicagel, and the like) is added to decrease relative air moisture for stabilisation of adhesion and content of active substances.

High voltage is inserted between the rod and the ground. The generator uses high-frequency switching technique which provides for consistent output voltage for preset values. Electronics provides for overcurrent protection of the generator and for protection against high voltage spark.

The nanomask prepared in this way in the form of multilayered sandwich is further cut into the final shape.

The last step in processing is assembly of the nanomask and wrapping it in coat which prevents entry of light and moisture and this way it stabilises content of active substances in the nanofibre layer because many active substances are both thermolabile and photolabile. Moreover, the whole nanomask is designed with the aim to dissolve on moist skin, therefore it is desirable to limit access of moisture to the nanomask to the minimum till the application. The coat of the nanomask also consists of several layers that are welded in spots so that the coat is air tight and non-transparent. The inside content is preserved with vacuum and possibly with moisture sorbents before the final closure. It is advantageous if the coat consists of aluminium foil preventing light entry, polyethylene (LDPE) protection the nanomask against entry of moisture and oxygen and polyethylene terephtalate (PETF).

### Summary of presented drawings

- Fig. 1: Illustration of scheme of cold electrostatic lamination; A Lamination device; B Lamination drum
- Fig. 2: Electron microscope snaps. Nano-fibre layer made of 10 wt.% solution of PVA 18-88; A magnification 5000x; B magnification 1000x; C magnification 5000x;
- Fig. 3: Electron microscope snaps. Nano-fibre layer made of 10 wt.% solution of PVA 18-88, according to example 1 B, magnification 1000x
- Fig. 4: Electron microscope snaps. Nano-fibre layer made of 10 wt.% solution of PVA 18-88, according to example 1 C, magnification 5000x
- Fig. 5: Possible cut-outs of nano-fibre sheets
- Fig. 6: Release of active substances from nanomask
- Fig. 7A: Emission spectrum of receptor solution - excitation wavelength 257 nm, according to example 6
- Fig. 7B: Excitation spectrum of vitamin A corresponding to the first peak in Fig. 7A, emission wavelength 420 nm
- Fig. 7C: Excitation spectrum of vitamin E corresponding to the second peak in Fig. 7A, emission wavelength 550 nm
- Fig. 7D: Emission spectrum of receptor solution taken after 5 minutes, 15 minutes and 30 minutes
- Fig. 7E: Development of intensity fluorescence of emission maximum of vitamin A at 385 nm and vitamin E (527 nm)
- Fig. 8: Permeation profile of model compound vitamin C through human skin during 14 hours according to example 7

### Examples of Invention Execution

### Example 1 A

### Nano-fibre sheet

100 g of PVA 18-88 with molar weight 130,000 g/mol was dissolved in 1000 ml of demineralized water in heated and homogeneously stirred reaction vessels at temperature 90°C, and under continuous stirring for 24 hours, and completely homogeneous 10 wt.% solution was formed. After complete dissolution of PVA, the solution was cooled progressively homogeneously under continuous stirring to temperature 25°C. Fibres were drawn electrostatically from the PVA solution using Nanospider TM (Elmarco) device under the following conditions: the fibre-forming electrode consisted of a string 0.3 mm thick with a die 0.6 mm and gap between the electrode and the counter-electrode set to 180 mm, frequency of move of the rider, and the cartridge with the fibre-forming solution was 4 s, voltage applied at fibre-forming electrode was +38 kV, voltage applied at the counter-electrode (collector) was -24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2 g/m². The specific weight was checked taking samples always in 1/3 of package 2 of the nano-fibre layer on the substrate nonwoven fabric, in half of package 2 and to the end of package 2 , always next to the right margin (3 specimens), in the middle (3 specimens), and next to the left margin (3 specimens). Specimens of size 10 x 10 cm were taken. The maximum standard deviation was 5%. The photos of the produced nano-fibre layer done with an electron microscope are presented in Figure 2.

### Example 1 B

### Nano-fibre sheet

100 g of PVA 18-88 with molar weight 130,000 g/mol was dissolved in 1000 ml of demineralized water in a 2 1 laboratory beaker at temperature 90°C, and under continuous stirring using magnetic stirring device for 24 hours and 10 wt.% solution was formed. After complete dissolution of PVA, the solution was cooled under continuous stirring to temperature 25°C. Fibres were drawn electrostatically from the PVA solution using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 5 s, voltage applied at fibre-forming electrode was +38 kV, voltage applied at the counter-electrode (collector) was -24 kV, draw-off speed of substrate nonwoven fabric 9 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The photos of the produced nano-fibre layer done with an electron microscope are presented in Figure 3, we can see drop-shaped defects in the nano-fibre layer.

### Example 1 C

### Nano-fibre sheet

100 g of PVA 18-88 with molar weight 130,000 g/mol was dissolved in 1000 ml of demineralized water in a 2 1 laboratory beaker at temperature 90°C, and under continuous stirring using magnetic stirring device for 24 hours and 10 wt.% solution was formed. After complete dissolution of PVA, the solution was cooled under continuous stirring to temperature 25°C. Fibres were drawn electrostatically from the PVA solution using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 3 s, voltage applied at fibre-forming electrode was +38 kV, voltage applied at the counter-electrode (collector) was -24 kV, draw-off speed of substrate nonwoven fabric 9 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2.4 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The photos of the produced nano-fibre layer done with an electron microscope are presented in Figure 4, we can see drop-shaped defects in the nano-fibre layer.

### Example 1 D

### Nano-fibre sheet containing hyaluronic acid,

95 g of polyvinylacetate was dissolved in 1000 ml of demineralized water in heated and homogeneously stirred reaction vessels at temperature 90°C, and under continuous stirring for 24 hours, and completely homogeneous 9.5 wt.% solution was formed. After complete dissolution of PVA, the solution was cooled homogeneously under continuous stirring to temperature 25°C, and 0.3 g of hyaluronic acid was added into the solution as the active substance. Fibres were drawn electrostatically from the solution of PVA and of hyaluronic acid using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 4 s, voltage applied at fibre-forming electrode was +38 kV, voltage applied at the counter-electrode (collector) was -24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 1.9 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The share of hyaluronic acid in the final sheet was 0.315 wt.%.

### Example 1 E

### Nano-fibre sheet containing gluconolactone

105 g of PVA 18-88 with molar weight 130,000 g/mol was dissolved in 1000 ml of demineralized water in heated and homogeneously stirred reaction vessels at temperature 90°C, and under continuous stirring for 24 hours, and completely homogeneous 10.5 weight % solution was formed. After complete dissolution of PVA, the solution was cooled homogeneously under continuous stirring to temperature 25°C, and 4.3 g of gluconolactone was added into the solution as the active substance. Fibres were drawn electrostatically from the solution of PVA and of gluconolactone using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 5 s, voltage applied at fibre-forming electrode was +38 kV, voltage applied at the counter-electrode (collector) was -24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2.1 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The share of gluconolactone in the final sheet was 3.846 wt.%.

### Example 1 F

### Nano-fibre sheet containing vitamins C and E

100 g of PVA 18-88 with molar weight 130,000 g/mol was dissolved in 1000 ml of demineralized water in heated and homogeneously stirred reaction vessels at temperature 90°C and under continuous stirring for 24 hours, and completely homogeneous 10 wt.% solution was formed. After complete dissolution of PVA, the solution was cooled homogeneously under continuous stirring to temperature 25°C and 5 g of vitamin C and 3.5 g of vitamin E was added into the solution as the active substance. Fibres were drawn electrostatically from the solution of PVA and of vitamins using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 4 s, voltage applied at fibre-forming electrode was +38 kV, voltage applied at the counter-electrode (collector) was - 24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The share of vitamin C in the final sheet was 4.61 wt.%. The share of vitamin E in the final sheet was 3.226 wt.%.

### Example 1 G

### Nano-fibre sheet containing vitamins B3 and B6.

105 g of PVA 18-88 with molar weight 130,000 g/mol was dissolved in 1000 ml of demineralized water in heated and homogeneously stirred reaction vessels at temperature 90°C and under continuous stirring for 24 hours, and completely homogeneous 10.5 wt.% solution was formed. After complete dissolution of PVA, the solution was cooled homogeneously under continuous stirring to temperature 25°C and 0.32 g of vitamin B3 and 0.4 g of vitamin B6 was added into the solution as the active substance. Fibres were drawn electrostatically from the solution of PVA and of vitamins using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 5 s, voltage applied at fibre-forming electrode was +38 kV, voltage applied at the counter-electrode (collector) was - 24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2.2 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The share of vitamin B3 in the final sheet was 0.303 wt.%. The share of vitamin B6 in the final sheet was 0.378 wt.%.

### Example 1 H

### Nano-fibre sheet containing salicylic acid and rose extract

100 g of PVA 18-88 with molar weight 130,000 g/mol was dissolved in 1000 ml of demineralized water in heated and homogeneously stirred reaction vessels at temperature 90°C and under continuous stirring for 24 hours, and completely homogeneous 10 wt.% solution was formed. After complete dissolution of PVA, the solution was cooled homogeneously under continuous stirring to temperature 25°C and 2 g of salicylic acid and 26 g of rose extract was added into the solution as the active substance. Fibres were drawn electrostatically from the solution of PVA and of salicylic acid and of rose extract using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 3 s, voltage applied at fibre-forming electrode was +38 kV, voltage applied at the counter-electrode (collector) was -24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The share of salicylic acid in the final sheet was 1.56 wt.%. The share of rose extract in the final sheet was 20.31 weight %.

### Example 1 I

### Nano-fibre sheet containing vitamins A a E.

93 g of PVA 18-88 with molar weight 130,000 g/mol was dissolved in 1000 ml of demineralized water in heated and homogeneously stirred reaction vessels at temperature 90°C and under continuous stirring for 24 hours, and completely homogeneous 10.5 wt.% solution was formed. After complete dissolution of PVA, the solution was cooled homogeneously under continuous stirring to temperature 25°C and 3.5 g of vitamin A a 3.5 g of vitamin E was added into the solution as the active substance. Fibres were drawn electrostatically from the solution of PVA and of vitamins using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 5 s, voltage applied at fibre-forming electrode was +38 kV, voltage applied at the counter-electrode (collector) was - 24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 2.2 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The share of vitamin A in the final sheet was 3.5 wt.%. The share of vitamin E in the final sheet was 3.5 wt.%.

### Example 1 J

### Nano-fibre sheet containing vitamin C

95 g of PVA 18-88 with molar weight 130,000 g/mol was dissolved in 1000 ml of demineralized water in heated and homogeneously stirred reaction vessels at temperature 90°C and under continuous stirring for 24 hours, and completely homogeneous 10.5 wt.% solution was formed. After complete dissolution of PVA, the solution was cooled homogeneously under continuous stirring to temperature 25°C and 5 g of vitamin C was added into the solution as the active substance. Fibres were drawn electrostatically from the solution of PVA and of vitamin C using Nanospider TM (Elmarco) device under the following conditions: the fibre-forming electrode consisted of a string 0.3 mm thick with a die 0.6 mm and gap between electrode and counter-electrode set to 180 mm, speed of the rider and the cartridge with the fibre-forming solution was 5 s, voltage applied at fibre-forming electrode was +38 kV, voltage applied at the counter-electrode (collector) was -24 kV, draw-off speed of substrate nonwoven fabric 11 mm/min. There was temperature 23°C and air moisture 35% inside the fibre-forming chamber.

Nanofibres were caught on substrate nonwoven fabric of polypropylene (polypropylene spunbond-Pegas Nonwovens) with specific weight 20 g/m², and the nano-fibre layer was applied on the nonwoven fabric with specific weight 10 g/m². The specific weight was checked taking samples always in 1/3 of package 2 of the nano-fibre layer on the substrate nonwoven fabric, in half of package 2 and to the end of package 2, always next to the right margin (3 specimens), in the middle (3 specimens), and next to the left margin (3 specimens). Specimens of size 10 x 10 cm were taken. The maximum standard deviation was 5%. The share of vitamin C in the final sheet was 5 wt.%.

### Example 2 A

### Cold electrostatic lamination

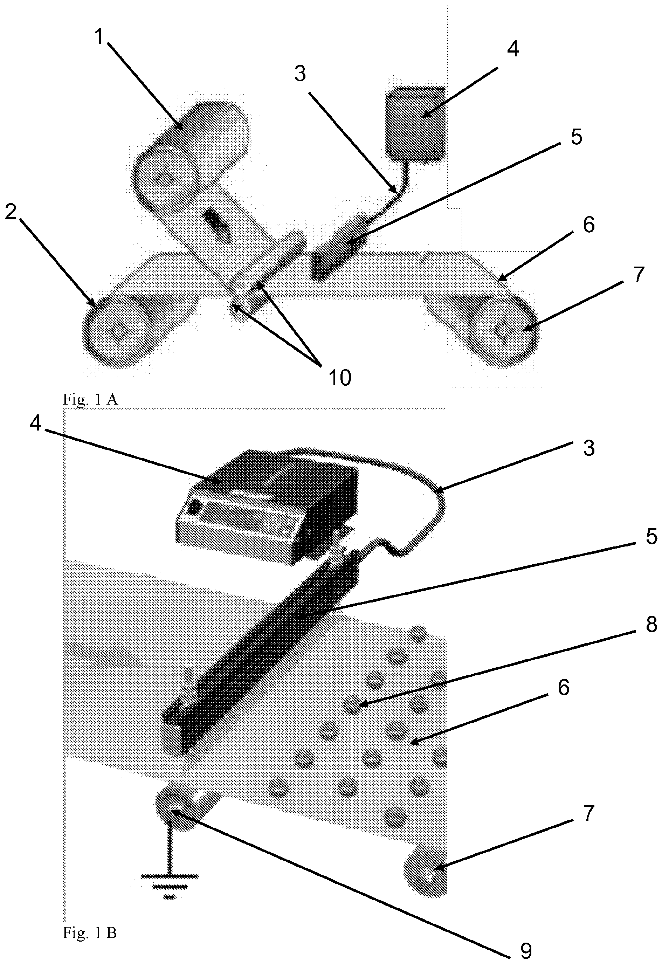

Nano-fibre sheet 2, wound on a paper roll and produced according to examples 1 A to J, and a roll of polyamide fabric 1 were inserted in the lamination machine in decoilers. Free ends of the nano-fibre sheet and of polyamide fabric were inserted between laminating calendering drums 10. The distance between the drums was set to 0.5 mm. The laminating machine involved equipment Simco Ion for static adjustment and this equipment created voltage from 20 kV to 30 kV with generator 1 and the voltage was inserted to charging rod 5 of the generator, and the rod was put from above on smoothed layers of nano-fibre sheet 2 and on polyamide fabrics 1 which passed lamination drums 10. The scheme of electrostatic lamination is presented in Figure 1. Voltage generator 4 was set to 20 kV and 4.3 mA and the positive charge was attached to the charging rod of generator 5 and the negative charge was attached to ground. Electrostatic lamination ran at 3.5 m/min, where the new laminated nano-fibre sheet 6 consisted of nonwoven antistatic polypropylene fabric, the nano-fibre layer was applied on it and dipoles of electrostatic forces pulled it to polyamide fabric 1. The positive ends of dipoles were oriented towards polyamide fabric and the negative ends to the nano-fibre layer. The produced laminated nano-fibre sheet 6 was wound on a paper roll of package 7 at 3.5 m/min.

### Example 2 B

### Thermal lamination

Nano-fibre sheet 2, wound on a paper roll and produced according to examples 1 D to J, and a roll of polyamide fabric 1 were inserted in the lamination machine in decoilers. Free ends of the nano-fibre sheet and of the polyamide fabric were inserted between heated laminating calendering drums 10. Glue EVA (ethylene vinyl-acetate) was applied on the whole area of polyamide fabric 1 with specific density 2 g/m². The distance between drums 10 was set to 0.5 mm. The lamination process ran at 3.5 m/min, and nano-fibre sheet 2 and polyamide fabric 1 were drawn over drum calenders 10 heated to 100°C. The new laminated nano-fibre sheet 6 consisted of nonwoven antistatic polypropylene fabric. The nano-fibre layer was applied on it and was covered with a stuck lamination layer. The new laminated nano-fibre sheet 6 was wound on a paper roll 7 at 1 m/min.

### Example 3

### Cutting out shapes from nano-fibre laminated sheets

The individual nanomasks, cover, neck cover, forehead coating, eye coating and coating of low neck were cut using a cutting template and a punch device from laminated nano-fibre sheets 6 wound on a paper roll 20 cm wide according to example 2.

Laminated nanofibre sheets 6 were placed at the position table of the punch device - cutter so that laminating PA fabric would be oriented upwards to the die. Dies of cylindrical shape were placed in the punch device one after another, punching with shear 0.1 mm with setting to thousandths of mm. 20 pieces of nanomasks, 45 pieces of cover, 40 pieces of neck cover, 45 pieces of forehead coating, 70 pieces of eye coating and 20 pieces of coating of low neck were produced of 1 m² of laminated nano-fibre sheets 6.

The shape of a nanomask, a cover, a neck cover, a forehead coating, an eye coating and a coating of low neck is presented in Figure 5. The area of a nanomask is 450 cm². The area of a cover is 220 cm². The area of a neck cover is 460 cm². The area of a forehead coating is 230 cm². The area of an eye coating is 220 cm². The area of a coating of low neck is 510 cm².

### Example 4

### Nano-fibre cosmetic cover packing

The shapes cut out from the sheets of electrostatically laminated nano-fibre cosmetic cover according to example 3 were wrapped into three-layer coat. A pocket was formed of LDPE, aluminium foil and PET foil in the following way: stripes with size 11 × 30 cm were cut of PET foil and aluminium foil and LDPE. The foils were placed one on another and the stripe was folded in half, a rectangle with size 11 × 15 cm was created, and the LDPE foil was inside and the aluminium foil was between LDPE and PET. 5mm rectangle rims were welded using laminating clamps at temperature 150°C from two opposite sides. This way a pocket with two opposite welded rims was created, one rim was formed through folding and one rim was free. Nanofibre cosmetic cover was folded and placed in the pocket together with a drying element through the free end. The free end was welded similarly, and in such a way not to exceed temperature 35°C in the sac. Nanofibre cosmetic cover wrapped into three-layer coat without access for air, moisture and light was created.

### Example 5

### Application of nanomask on skin

Volunteers' skin was cleaned from dirt and make-up with cosmetic tonic and moistened with 3 ml of water using a cosmetic tampon. Nanomasks produced of nano-fibre sheets according to examples 1 D to J and then laminated according to example 2 A were unfolded carefully, lamination fabric was stripped off them and in case of masks laminated according to example 2B, nonwoven fabric was stripped off. Then they were applied to the volunteers' moistened skin, and contact occurred between the water-soluble carrier PVA containing active substance and the moistened skin. Each nanomask was tested with three volunteers. The nanomask was kept on the skin of each of the volunteers for 8 minutes. After 8 minutes most of water-soluble polymer carrier was dissolved and only nonwoven fabric, or PA lamination layer according to example 2 B with residua of carrier kept on the skin. It was used to rub down residua of the nanomask from the face. Nonwoven fabric and lamination fabric with a minimum residuum of the nanomask was subjected to analysis of residual active substances. HPLC showed that after 8 minutes of application, the nanomask transfers 92 to 95% of active substance contained in the sheet to the skin. HPLC also showed that after cold electrostatic lamination 87 to 92% of the original active substance keeps in a nano-fibre sheet, compared with common thermal lamination, where not more than 5% of the original active substance keeps in a nano-fibre sheet. The content of active substance after individual production steps is presented in Figure 6.

### Example 6

### Measurement of release kinetics of active substances from nano-fibre sheet and measurement of diffusion coefficient with nitrocellulose membrane

The determination of relative kinetics of release of active substances and determination of diffusion coefficient was performed using fluorescence analysis. Therefore a nano-fibre sheet containing active substances showing fluorescence - vitamins A a E, the nano-fibre sheet according to example 1 I, was selected for this experiment. The active layer of the nano-fibre sheet according to example 1 I with area 10 cm² was separated from the lamination layer and the layer of nonwoven fabric and it was dissolved under continuous stirring in 10 ml of purified water. This way prepared solution was used as stock solution for the diffusion experiment. The diffusion experiment was carried out in a standard Franz diffusion cell - vertical diffusion apparatus with two compartment - for stock solution and for receptor solution. Both the compartments are separated with a porous diffusion barrier. 2.5 ml of stock solution, nitrocelullose ultrafiltration membrane Pragopor (diameter 50 mm, size of pores 0.23 µm) and 25 ml of receptor solution was used for the experiment. The receptor solution was phosphate buffer (PBS, pH = 7.4) which filled all the space of the compartment for receptor solution. Specimens of receptor solution were taken after 5 minutes, 15 minutes and 30 minutes and their fluorescence spectrum was measured (fluorescence spectrophotometer FS 5, Edinburgh Instruments, excitation wavelength 257 nm).

Fluorescence spectra in Figures 7A to 7E show release of vitamins A a E. Figure 7A shows the emission spectrum of the receptor solution after 5 minutes with two separated peaks corresponding two fluorescence substances - vitamin A with maximum of fluorescence intensity at 385 nm, vitamin E with maximum of fluorescence intensity at 527 nm. The excitation spectra of the substances are shown in Figures 7B and 7C. Figures 7D and 7E show kinetics of vitamins release in different time.

It is evident that the main part of the active substances is transported through the membrane within the first 5 minutes (more than 60% of the total amount transported in 30 minutes). Transport of vitamin A within 15 minutes corresponds to 90% of the total transported vitamin A, for vitamin E it is 98%.

### Example 7

### Measurement of release kinetics of active substances from nano-fibre sheet and measurement of diffusion coefficient with human skin

The determination of release kinetics of active substances and determination of diffusion coefficient with human skin was performed using liquid chromatography. The active layer of the nano-fibre sheet produced according to example 1 J with area 2 cm² was separated from the lamination layer. The diffusion experiment was carried out in a standard Franz diffusion cell - vertical diffusion apparatus with two compartment - for the specimen and for the receptor solution. The both compartment are separated with a porous diffusion barrier. 2 cm² of the nano-fibre sheet, human skin (abdominal skin) and 25 ml of the receptor solution were used for the experiment. The receptor solution was phosphate buffer (PBS, pH = 7.4) and it filled all the space of the compartment for receptor solution. The nano-fibre sheet specimen was damped with 0.3 ml of purified water and laid with its active layer on human skin into the Franz cell. The specimens of the receptor solution were taken each two hours, the last collection took place after 14 hours. Liquid chromatography (HPLC) was used for detection of the active substances in the receptor solution.

For the sake of a comparison, hydrogel with the same content of vitamin C (5 wt.%) was analysed similarly.

The results are presented in Figure 8 and show that the specimen of the nano-fibre cover has reached 270% permeation after 14 hours in comparison with with hydrogel applied under identical conditions.

### List of marks for terms

- 1.: Package of PA fabric
- 2.: Package of nano-fibre layer on substrate nonwoven fabric
- 3.: Guide
- 4.: D.C. high voltage generator
- 5.: Charging electrode
- 6.: Laminated nano-fibre sheet
- 7.: Package of laminated nano-fibre sheet 6
- 8.: Dipole
- 9.: Ground
- 10.: Laminating calendering drum

Applicability in Industry

Cosmetics, medicine, aesthetic medicine.

## Claims

1. An electrostatically laminated nanofibre cosmetic cover with a removable lamination layer **characterised by** the fact that it comprises an antistatic substrate layer nonwoven fabric, a nano-fibre layer of water-soluble polymer and a non-conducting synthetic lamination fabric, which is the removable lamination layer, wherein the nano-fibre layer of water-soluble polymer and the non-conducting synthetic lamination fabric are polarized concerning orientation, and therefore they pull one another due to opposite-oriented dipoles.

2. The electrostatically laminated nanofibre cosmetic cover with the removable lamination layer according to claim 1 **characterised by** the fact that the water-soluble polymer is selected in this group: polyvinyl alcohol, polyvinyl acetate, polycaprolactone, polydioxanone, polyethylene oxide, polyhydroxibutyrate, polyvinyl butyrate, polyvinyl butyral, polylactide, polylactide-co-caprolaktone, polylactide-co-glycolide, polylactide-co-caprolactone-co-glycolide, polyvinyl alcohol , polyvinyl pyrrolidone, elastin, collagen, gelatine, zein, wheat protein, polysaccharides and their copolymers, salts, acids; or their mixtures.

3. The electrostatically laminated nanofibre cosmetic cover with the removable lamination layer according to claim 2 **characterised by** the fact that the polysaccharides and their copolymers, salts, acids are selected in this group: cellulose acetate, sodium alginate, hyaluronic acid, chitosan, carboxymethylchitosan, chitin, hyaluronans, glucans.

4. The electrostatically laminated nanofibre cosmetic cover with the removable lamination layer according to claim 1 **characterised by** the fact that the nonwoven fabric is made of polypropylene or antistatically adjusted polyamide.

5. The electrostatically laminated nanofibre cosmetic cover with the removable lamination layer according to claim 1 **characterised by** the fact that the non-conducting synthetic lamination fabric is made of polyamide or silicone paper.

6. The electrostatically laminated nanofibre cosmetic cover with the removable lamination layer according to claim 1 **characterised by** the fact that the nano-fibre layer of water-soluble polymer has specific weight 1.5 to 10 g/m².

7. The electrostatically laminated nanofibre cosmetic cover with the removable lamination layer according to claim 1 **characterised by** the fact that the nano-fibre layer of water-soluble polymer contains 0.001 to 25 wt.% of an active substance or their mixture.

8. The electrostatically laminated nanofibre cosmetic cover with the removable lamination layer according to claim 7 **characterised by** the fact that the active substance is selected in this group: vitamin C, mineral salt of ascorbic acid, vitamin A, retinyl palmitate, ester of retinol and of palmitic acid, their salt or derivative; vitamin E, ester of acetic acid and of tocopherol, their derivative and salt; gluconolactone, azelaic acid, salicylic acid, benzoyl peroxide, maltobionic acid, N-acetyl tyrosamine, etylferulate, hydroxymethylurea, Ajidew, methylprotocatechic acid, cetearyl etylhexanoate, bisabol, sodium fluoride, collagen, vitamin B1, its salt or derivative; triclosan, cetrimonium chloride, quarternary ammonium compound, Lumiskin, askorbyl glucoside, sodium cocoylisethionate, cocamidopropyle dimethylamine, function lipid, dimethicone, ester of tocopherol, mineral salts of ascorbic acid, biotin, erythrulose, stearate, ferulic acid, zinc oxide in cocos alkanes or their mixture.

9. The electrostatically laminated nanofibre cosmetic cover with the removable lamination layer according to claim 7 **characterised by** the fact that the nano-fibre layer of water-soluble polymer contains 0.1 to 5 wt.% of an active substance, and the active substance is panthenol or alcohol analogue of panthotenic acid and/or the nano-fibrelayer contains 0.001 to 3 wt.% of an active substance, and the active substance is vitamin B3, vitamin B6, riboflavin sodium phosphate, its salt or derivative; hyaluronic acid , its derivative or salt and/or the nano-fibre layer contains 0.1 to 8 wt.% of an active substance, and the active substance is Arbutin or its derivative and/or the nano-fibre layer contains 0.01 to 25 wt.% of a natural extract or extracts.

10. The electrostatically laminated nanofibre cosmetic cover with the removable lamination layer according to claim 1 **characterised by** the fact that it is wrapped into a multi-layer coating.

11. The electrostatically laminated nanofibre cosmetic cover with the removable lamination layer according to claim 10 **characterised by** the fact that the multi-layer coating is composed of low-density polyethylene, of polyethylene terephthalate and of aluminium.

12. A method for lamination of nano-fibre cosmetic cover **characterised by** the fact that the non-conducting synthetic lamination foil and the nano-fibre sheet comprising the nano-fibre layer applied on the substrate layer of nonwoven fabric are inserted between roller calenders, and the nano-fibre sheet is oriented with the nano-fibre layer towards the non-conducting synthetic lamination foil, calendering drums are actuated on outstanding smoothed layers of the nano-fibre sheet and the non-conducting synthetic lamination foil, D.C. voltage 20 to 70 kV, is applied from the calendering drums in the whole area and this way formed the laminated nanofibre cover is wound with speed 2 to 5 m/min.

13. The method for lamination of nano-fibre cosmetic cover according to claim 12 **characterised by** the fact that the D.C. voltage is at most 30 kV and electric current is at most 5 mA.

14. Use of electrostatically laminated nanofibre cosmetic cover with the removable lamination layer according to claim 1 as a facial mask.

## Patentansprüche

1. Elektrostatisch kaschierte Nanofaser-Kosmetikabdeckung mit einer entfernbaren Kaschierschicht, **dadurch gekennzeichnet, dass** er ein antistatisches Trägerschichtvlies, eine Nanofaserschicht aus wasserlöslichem Polymer und ein nichtleitendes synthetisches Kaschiergewebe, das die entfernbare Kaschierschicht ist, umfasst, wobei die Nanofaserschicht aus wasserlöslichem Polymer und das nichtleitende synthetische Kaschiergewebe hinsichtlich der Orientierung polarisiert sind und sich daher aufgrund entgegengesetzt orientierter Dipole gegenseitig anziehen.

2. Elektrostatisch kaschierte Nanofaser-Kosmetikabdeckung mit der entfernbaren Kaschierschicht nach Anspruch 1, **dadurch gekennzeichnet, dass** das wasserlösliche Polymer aus dieser Gruppe ausgewählt ist: Polyvinylalkohol, Polyvinylacetat, Polycaprolacton, Polydioxanon, Polyethylenoxid, Polyhydroxibutyrat, Polyvinylbutyrat, Polyvinylbutyral, Polylactid, Polylactid-co-caprolakton, Polylactid-co-glycolid, Polylactid-co-caprolacton-co-glycolid, Polyvinylalkohol, Polyvinylpyrrolidon, Elastin, Kollagen, Gelatine, Zein, Weizenprotein, Polysaccharide und deren Copolymere, Salze, Säuren; oder deren Mischungen.

3. Elektrostatisch kaschierte Nanofaser-Kosmetikabdeckung mit der entfernbaren Kaschierschicht nach Anspruch 2, **dadurch gekennzeichnet, dass** die Polysaccharide und deren Copolymere, Salze, Säuren aus dieser Gruppe ausgewählt sind: Celluloseacetat, Natriumalginat, Hyaluronsäure, Chitosan, Carboxymethylchitosan, Chitin, Hyaluronane, Glucane.

4. Elektrostatisch kaschierte Nanofaser-Kosmetikabdeckung mit der entfernbaren Kaschierschicht nach Anspruch 1, **dadurch gekennzeichnet, dass** der Vliesstoff aus Polypropylen oder antistatisch behandeltem Polyamid besteht.

5. Elektrostatisch kaschierte Nanofaser-Kosmetikabdeckung mit der entfernbaren Kaschierschicht nach Anspruch 1, **dadurch gekennzeichnet, dass** das nichtleitende synthetische Kaschiergewebe aus Polyamid- oder Silikonpapier besteht.

6. Elektrostatisch kaschierte Nanofaser-Kosmetikabdeckung mit der entfernbaren Kaschierschicht nach Anspruch 1, **dadurch gekennzeichnet, dass** die Nanofaserschicht aus wasserlöslichem Polymer ein spezifisches Gewicht von 1,5 bis 10 g/m² aufweist.

7. Elektrostatisch kaschierte Nanofaser-Kosmetikabdeckung mit der entfernbaren Kaschierschicht nach Anspruch 1, **dadurch gekennzeichnet, dass** die Nanofaserschicht aus wasserlöslichem Polymer 0,001 bis 25 Gew.-% eines Wirkstoffs oder seiner Mischung enthält.

8. Elektrostatisch kaschierte Nanofaser-Kosmetikabdeckung mit der entfernbaren Kaschierschicht nach Anspruch 7, **dadurch gekennzeichnet, dass** der Wirkstoff aus dieser Gruppe ausgewählt ist: Vitamin C, Mineralsalz der Ascorbinsäure, Vitamin A, Retinylpalmitat, Ester von Retinol und Palmitinsäure, deren Salz oder Derivat; Vitamin E, Ester der Essigsäure und von Tocopherol, deren Derivat und Salz; Gluconolacton, Azelainsäure, Salicylsäure, Benzoylperoxid, Maltobionsäure, N-Acetyltyrosamin, Etylferulat, Hydroxymethylharnstoff, Ajidew, Methylprotocatechinsäure, Cetearyl-Etylhexanoat, Bisabol, Natriumfluorid, Kollagen, Vitamin B1, dessen Salz oder Derivat; Triclosan, Cetrimoniumchlorid, quartäre Ammoniumverbindung, Lumiskin, Askorbylglucosid, Natriumcoylisethionat, Cocamidopropyldimethylamin, Funktionslipid, Dimethicon, Ester von Tocopherol, Mineralsalze der Ascorbinsäure, Biotin, Erythrulose, Stearat, Ferulinsäure, Zinkoxid in Kokosalkanen oder deren Mischung.

9. Elektrostatisch kaschierte Nanofaser-Kosmetikabdeckung mit der entfernbaren Kaschierschicht nach Anspruch 7, **dadurch gekennzeichnet, dass** die Nanofaserschicht aus wasserlöslichem Polymer 0,1 bis 5 Gew.-% eines Wirkstoffs enthält und der Wirkstoff Panthenol oder Alkoholanalogon der Panthotensäure ist und/oder die Nanofaserschicht 0,001 bis 3 Gew.-% eines Wirkstoffs enthält und der Wirkstoff Vitamin B3, Vitamin B6, Riboflavin-Natriumphosphat, dessen Salz oder Derivat ist, Hyaluronsäure, dessen Derivat oder Salz und/oder die Nanofaserschicht 0,1 bis 8 Gew.-% eines Wirkstoffs enthält und der Wirkstoff Arbutin oder dessen Derivat ist und/oder die Nanofaserschicht 0,01 bis 25 Gew.-% eines natürlichen Extrakts oder natürlicher Extrakte enthält.

10. Elektrostatisch kaschierte Nanofaser-Kosmetikabdeckung mit der entfernbaren Kaschierschicht nach Anspruch 1, **dadurch gekennzeichnet, dass** er in eine mehrlagige Beschichtung eingehüllt ist.

11. Elektrostatisch kaschierte Nanofaser-Kosmetikabdeckung mit der entfernbaren Kaschierschicht nach Anspruch 10, **dadurch gekennzeichnet, dass** die mehrlagige Beschichtung aus Polyethylen niedriger Dichte, aus Polyethylenterephthalat und aus Aluminium besteht.

12. Verfahren zur Kaschierung einer Nanofaser-Kosmetikabdeckung, **dadurch gekennzeichnet, dass** die nichtleitende synthetische Kaschierfolie und das Nanofaserblatt, das die auf die Trägerschicht aus Vliesstoff aufgebrachte Nanofaserlage umfasst, zwischen Walzenkalander eingelegt werden und das Nanofaserblatt mit der Nanofaserlage zur nichtleitenden synthetischen Kaschierfolie ausgerichtet wird, Kalandertrommeln auf herausragenden geglätteten Schichten des Nanofaserblattes und die nichtleitende synthetische Kaschierfolie einwirken, Gleichspannung von 20 bis 70 kV von den Kalandertrommeln im gesamten Bereich angelegt wird und so der kaschierte Nanofaserbezug mit einer Geschwindigkeit von 2 bis 5 m/min gewickelt wird.

13. Verfahren zum Kaschieren einer Nanofaser-Kosmetikabdeckung nach Anspruch 12, **dadurch gekennzeichnet, dass** die Gleichspannung höchstens 30 kV und der elektrische Strom höchstens 5 mA beträgt.

14. Verwendung einer elektrostatisch kaschierten Nanofaser-Kosmetikabdeckung mit der entfernbaren Kaschierschicht nach Anspruch 1 als Gesichtsmaske.

## Revendications

1. Couverture cosmétique en nanofibre stratifiée électrostatiquement avec une couche de stratification amovible **caractérisée en ce qu'**elle comprend une couche de substrat antistatique de tissu non tissé, une couche nanofibre de polymère hydrosoluble et un tissu de stratification synthétique non conducteur, qui est la couche de stratification amovible, la couche nanofibre de polymère hydrosoluble et le tissu de stratification synthétique non conducteur étant polarisés en ce qui concerne l'orientation, et donc ils tirent l'un l'autre en raison de dipôles orientés de manière opposée.

2. Couverture cosmétique en nanofibre stratifiée électrostatiquement avec la couche de stratification amovible selon la revendication 1, **caractérisée en ce que** le polymère hydrosoluble est choisi dans le groupe : alcool polyvinylique, acétate de polyvinyle, polycaprolactone, polydioxanone, polyoxyde d'éthylène, butyrate de polyvinyle, butyral de polyvinyle, polylactide, polylactide-co-caprolactone, polylactide-co-glycolide, polylactide-co-caprolactone-co-glycolide, alcool polyvinylique, polyvinylpyrrolidone, élastine, collagène, gélatine, zéine, protéines de blé, polysaccharides et leurs copolymères, sels, acides; ou leurs mélanges.

3. Couverture cosmétique en nanofibre stratifiée électrostatiquement avec la couche de stratification amovible selon la revendication 2, **caractérisée en ce que** les polysaccharides et leurs copolymères, sels, acides sont choisis dans le groupe : acétate de cellulose, alginate de sodium, acide hyaluronique, chitosane, carboxyméthylchitosane, chitine, hyaluronanes, glucanes.

4. Couverture cosmétique en nanofibre stratifiée électrostatiquement avec la couche de stratification amovible selon la revendication 1, **caractérisée en ce que** le tissu non-tissé est en polypropylène ou en polyamide avec traitement antistatiquement.

5. Couverture cosmétique en nanofibre stratifiée électrostatiquement avec la couche de stratification amovible selon la revendication 1, **caractérisée en ce que** le tissu de stratification synthétique non conducteur est en papier polyamide ou silicone.

6. Couverture cosmétique en nanofibre stratifiée électrostatiquement avec la couche de stratification amovible selon la revendication 1, **caractérisée en ce que** la couche de nanofibre en polymère hydrosoluble a un poids spécifique de 1,5 à 10 g/m².

7. Couverture cosmétique en nanofibre stratifiée électrostatiquement avec la couche de stratification amovible selon la revendication 1, **caractérisée en ce que** la couche de nanofibre en polymère hydrosoluble contient 0,001 à 25% en poids d'une substance active ou de leur mélange.

8. Couverture cosmétique en nanofibre stratifiée électrostatiquement avec la couche de stratification amovible selon la revendication 7, **caractérisée en ce que** la substance active est choisie dans le groupe : vitamine C, ascorbate minéral, vitamine A, palmitate de rétinyle, ester de rétinol et de l'acide palmitique, leur sel ou dérivé; vitamine E, ester de l'acide acétique et du tocophérol, leur dérivé et sel; gluconolactone, acide azélaïque, acide salicylique, peroxyde de benzoyle, acide maltobionique, N-acétyl tyrosamine, éthyl férulate, hydroxyméthylurée, Ajidew, acide méthylprotocatéchique, cétéaryle ethylhexanoate, bisabol, fluorure de sodium, collagène, vitamine B1, son sel ou dérivé; triclosan, chlorure de cétrimonium, composé d'ammonium quartenaire, Lumiskin, ascorbyl glucoside, cocoyl iséthionate de sodium, cocamidopropyl diméthylamine, lipide fonctionnel, diméthicone, ester de tocophérol, sels minéraux de l'acide ascorbique, biotine, érythrulose, stéarate, acide férulique, oxyde de zinc dans les alcanes de cocos ou leur mélange.

9. Couverture cosmétique en nanofibre stratifiée électrostatiquement avec la couche de stratification amovible selon la revendication 7, **caractérisée en ce que** la couche de nanofibre en polymère hydrosoluble contient 0,1 à 5% en poids d'une substance active, et que la substance active est le panthénol ou l'alcool analogue de l'acide panthotène et/ou la couche de nanofibre contient 0,001 à 3% en poids d'une substance active, et que la substance active est la vitamine B3, la vitamine B6, le phosphate de sodium de riboflavine, son sel ou dérivé; l'acide hyaluronique, son dérivé ou sel et/ou la couche de nanofibre contient 0,1 à 8% en poids d'une substance active, et la substance active est l'arbutine ou son dérivé et/ou la couche de nanofibre contient 0,01 à 25% en poids d'un ou des extraits naturels.

10. Couverture cosmétique en nanofibre stratifiée électrostatiquement avec la couche de stratification amovible selon la revendication 1, **caractérisée en ce qu'**elle est enveloppée dans un revêtement multicouche.

11. Couverture cosmétique en nanofibre stratifiée électrostatiquement avec la couche de stratification amovible selon la revendication 10, **caractérisée en ce que** le revêtement multicouche est composé de polyéthylène basse densité, de polyéthylène téréphtalate et d'aluminium.

12. Procédé de stratification d'un couverture cosmétique en nanofibre **caractérisé en ce que** la feuille de stratification synthétique non conductrice et la feuille de nanofibre comprenant la couche de nanofibre appliquée sur la couche de substrat en tissu non tissé sont insérées entre des calandres de laminage, et que la feuille de nanofibre est orientée avec la couche de nanofibre vers la feuille de stratification synthétique non conductrice, que des cylindres de calandrage sont actionnés sur des couches lissées saillants de la feuille de nanofibre et que la feuille de stratification synthétique non conductrice, tension électrique continue de 20 à 70 kV, est appliquée à partir cylindres de calandrage dans toute la zone et que de cette façon, le revêtement de nanofibre stratifié est enroulé avec une vitesse de 2 à 5 m/min.

13. Procédé de stratification d'une couverture cosmétique en nanofibre selon la revendication 12 **caractérisé en ce que** la tension électrique continue est d'au plus 30 kV et le courant électrique d'au plus 5 mA.

14. Utilisation d'une couverture cosmétique en nanofibre stratifiée électrostatiquement avec la couche de stratification amovible selon la revendication 1 comme masque facial.
